# EUROPÄISCHE PATENTSCHRIFT

(11) **EP 3 185 965 B1**
(45) Veröffentlichungstag und Bekanntmachung des Hinweises auf die Patenterteilung: **23.10.2019**
(21) Anmeldenummer: 15749809.8
(22) Anmeldetag: 12.08.2015
(51) Int. Cl.: A61Q 5/10, A61K 8/22

(54) **MULTITONALE EINSCHRITT-FÄRBUNGEN MIT VERDICKTER VORBEHANDLUNGSLÖSUNG II**
MULTI-TONAL ONE STEP DYEING PROCESS USING A THICKENED PRETREATMENT SOLUTION II
COLORATION MULTITON EN UNE ÉTAPE UTILISANT UN AGENT DE PRÉ-TRAITEMENT ÉPAISSI II

(30) Priorität: 26.08.2014 DE 102014216943
(43) Veröffentlichungstag der Anmeldung: 05.07.2017
(73) Patentinhaber: Henkel AG & Co. KGaA, 40589 Düsseldorf (DE)
(72) Erfinder: NEUBA, Constanze, 41516 Grevenbroich (DE); MÜLLER, Burkhard, 40221 Düsseldorf (DE)
(86) Internationale Anmeldenummer: PCT/EP2015/068564
(87) Internationale Veröffentlichungsnummer: WO 2016/030191

(56) Entgegenhaltungen:
- WO-A2-01/15662

## Beschreibung

Gegenstand der vorliegenden Anmeldung ist ein Verfahren zur Behandlung keratinischer Fasern, welches es erlaubt, in einem Färbeschritt das Haar zu colorieren und gleichzeitig eine multitonale Färbung mit helleren ("highlights") oder dunkleren ("lowlights") Partien (Strähnchen) zu erzeugen. Hierzu wird eine verdickte Farbstoffvorbehandlungslösung mit direktziehenden Farbstoffen, welche keine Oxidationsfarbstoffvorprodukte vom Entwickler- und Kupplertyp enthält, in Kombination mit einem oxidativem Färbemittel verwendet.

Im Laufe der Zeit und insbesondere unter Einwirkung von äußeren Einflüssen wie Licht oder atmosphärischen Schadstoffen verliert oder verändert das Haar seine natürliche Farbe und seinen Glanz bzw. Schimmer. Aus diesem Grund finden Haarfärbemittel breite Anwendung, welche entweder im Friseurbereich oder durch die Heimanwendung genutzt werden.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, welche unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, welche als färbende Komponente sogenannte direktziehende Farbstoffe ("Direktzieher") enthalten.

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen Substrate aufgehellt oder gar gebleicht werden, werden die das Substrat färbenden Farbstoffe meist oxidativ unter Einsatz von entsprechenden Oxidationsmitteln, wie Wasserstoffperoxid, entfärbt.

Bei der Haarfärbung - insbesondere bei der Haarfärbung durch die Heimanwendung - tritt das Problem auf, dass natürliche Farbnuancierungen vollständig überdeckt werden, so dass multitonale Färbungen schwer zu realisieren sind.

Um dem Haar ein natürlicheres Aussehen zu verleihen, ist es im Stand der Technik bekannt, gefärbte Haare durch die gezielte Anwendung von Oxidationsmitteln partiell zu entfärben. Die Haarpartien ("Strähnchen"), auf welche die Oxidationsmittel aufgetragen werden, bleichen dabei mindestens anteilsweise aus, woraus eine multitonale Haarfarbe resultiert. Die Applikation des Oxidationsmittels erfolgt dabei mit einer Bürste oder einem Pinsel, wobei die nicht zu behandelnden Haare gegebenenfalls mit Aluminiumfolie oder einer sogenannten "Strähnchenhaube" vor der Entfärbung geschützt werden.

Diese Applikationsart löst zwar das Problem der möglichst natürlichen Färbung von Haaren, lässt aber nur das Setzen von "highlights" zu. Für "lowlights", d. h. dunklere Partien, müsste nachgefärbt werden. In den zuvor beschriebenen Fällen wäre daher ein zeitaufwendiger zweiter Entfärbe- bzw. Färbeschritt erforderlich, welcher sich an die erste Färbung anschließt. Insbesondere bei der Heimanwendung müsste daher zunächst das gesamte Haar coloriert werden, bevor die Anwenderin "highlights" oder "lowlights" setzen kann. Von vielen Verbraucherinnen wird dies als zu zeitaufwendig und auch als frustrierend empfunden, da der wesentliche farbverändernde Schritt anfangs erfolgt und in einem zweiten Schritt lediglich "korrigiert" wird.

Die WO 01/15662 A1 offenbart ein Verfahren zum Färben keratinischer Fasern, bei dem in einem ersten Schritt ein Vorbehandlungsmittel auf die Fasern aufgetragen wird, das mindestens einen direktziehenden Farbstoff enthält, und in einem zweiten Schritt die Fasern einer herkömmlichen Färbung mit mindestens einem synthetischen Farbstoff(vorprodukt) unterzogen werden.

Der vorliegenden Erfindung lag die Aufgabe zugrunde, ein Verfahren bereitzustellen, welches multitonale Färbungen in einem Färbeschritt ermöglicht. Dabei sollte die Färbung des Haares mit der Erzeugung von "highlights" oder "lowlights" einhergehen, damit nach dem Ausspülen des Färbemittels direkt ein Ergebnis sichtbar ist.

Es wurde nun gefunden, dass eine partielle Vorbehandlung von Faserarealen bzw. Strähnchen dazu führt, dass diese Areal bzw. Strähnchen später intensiver oder weniger intensiv angefärbt werden. Durch die Vorpenetration bzw. Vorbehandlung einzelner Faserareale bzw. Strähnchen färbt das unmittelbar darauffolgend angewendete Färbemittel das Haar multitonal und es wird direkt nach dem Färbeschritt ein natürliches Färbeergebnis mit "highlights" oder "lowlights" erhalten.

Ein Gegenstand der vorliegenden Erfindung ist daher ein Verfahren zum oxidativen Färben von keratinischen Fasern, wobei das Verfahren die folgenden Verfahrensschritte in der angegebenen Reihenfolge umfasst:
a) Applikation eines kosmetischen Mittels (M1) auf die keratinischen Fasern, wobei das kosmetische Mittel (M1) nur auf einzelne Strähnchen appliziert wird,
b) Einwirkenlassen des Mittels (M1) für einen Zeitraum von 30 Sekunden bis 30 Minuten auf den keratinischen Fasern,
c) Applikation eines kosmetischen Mittels (M2) auf die mit dem kosmetischen Mittel (M1) beaufschlagten keratinischen Fasern,
d) Einwirkenlassen der kosmetischen Mittel (M1) und (M2) für einen Zeitraum von 1 bis 70 Minuten auf den keratinischen Fasern,
e) Ausspülen der kosmetischen Mittel (M1) und (M2),
dadurch gekennzeichnet, dass
- das kosmetische Mittel (M1)
   mindestens einen direktziehenden Farbstoff enthält (M1-1),
   mindestens ein Verdickungsmittel enthält (M1-2), und
   keine Oxidationsfarbstoffvorprodukte enthält (M1-3)
   einen pH-Wert von 9,2 bis 10,5 aufweist,
   eine dynamische Viskosität von 10.000 bis 50.000 mPa*s, gemessen mit Brookfield RDV II+, Spindel Nr. 4, 4 rpm, 20 °C, aufweist,
- das kosmetische Mittel (M2)
   mindestens ein Oxidationsfarbstoffvorprodukt enthält (M2-1), und
   mindestens ein Oxidationsmittel enthält (M2-2),
- das kosmetischen Mittel (M1) und das kosmetischen Mittel (M2) identische pH-Werte aufweisen.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn das erfindungsgemäße Verfahren zur Färbung von menschlichen Haaren verwendet wird.

Weiterhin sind unter dem Begriff "Verdickungsmittel" im Rahmen der vorliegenden Erfindung Verbindungen zu verstehen, welche Flüssigkeiten, insbesondere Wasser, binden können und die Viskosität dieser Flüssigkeiten erhöhen. Hierzu gehören im Rahmen der vorliegenden Erfindung auch Gelbildner, welche in der Lage sind, Flüssigkeiten zu Zusammensetzungen mit gelartiger Konsistenz bzw. zu Gelen zu verdicken. Unter gelartigen kosmetischen Mitteln bzw. Gelen werden erfindungsgemäß formbeständige, leicht deformierbare disperse Systeme aus mindestens zwei Komponenten, dem Gelbildner (meist ein fester, kolloidzerteilter Stoff mit langen oder stark verzweigten Verbindungen) und einer Flüssigkeit (meist Wasser) als Dispersionsmittel, verstanden. Der Gelbildner bildet in der Flüssigkeit ein räumliches Netzwerk aus, wobei die einzelnen gelbildenden Verbindungen durch Haupt- und/oder Nebenvalenzen an verschiedenen räumlichen Punkten untereinander aneinanderhaften.

Erfindungsgemäß bevorzugt werden die Verfahrensschritte a) bis e) in der zuvor angeführten Reihenfolge mit einem Zeitabstand zwischen den einzelnen Verfahrensschritten von jeweils 0 bis 60 Minuten, vorzugsweise von jeweils 0 bis 40 Minuten, insbesondere von jeweils 0 bis 30 Minuten, durchgeführt.

Im ersten Verfahrensschritt (Verfahrensschritt a)) des erfindungsgemäßen Verfahrens wird ein kosmetisches Mittel (M1) auf die Fasern appliziert. Dieses kosmetische Mittel (M1), welches nachfolgend auch als Vorbehandlungsmittel oder als Vorpenetrationsmittel bezeichnet wird, wird über einen Zeitraum von 30 Sekunden bis 30 Minuten auf den keratinischen Fasern belassen (Schritt b) des erfindungsgemäßen Verfahrens).
Erfindungsgemäß bevorzugt sind jedoch eher kürzere Einwirkzeiten des Vorbehandlungsmittels. Besonders bevorzugte erfindungsgemäße Verfahren sind dadurch gekennzeichnet, dass das kosmetische Mittel (M1) in Verfahrensschritt b) für einen Zeitraum von 30 Sekunden bis 30 Minuten, vorzugsweise von 30 Sekunden bis 20 Minuten, bevorzugt von 30 Sekunden bis 18 Minuten, insbesondere von 30 Sekunden bis 15 Minuten, auf den keratinischen Fasern Einwirken gelassen wird.

Die Vorbehandlung von keratinischen Fasern mit dem kosmetischen Mittel (M1) führt dazu, dass an diesen Stellen bereits Inhaltsstoffe des Vorbehandlungsmittels (M1) an den keratinischen Fasern haften bzw. in die keratinischen Fasern eingedrungen sind, so dass das Färbeergebnis bei anschließendem Auftragen des kosmetischen Mittels (M2) an diesen Stellen verstärkt bzw. aufgehellt wird. Auf diese Weise kann in einem Färbeschritt das Haar coloriert und gleichzeitig eine multitonale Färbung mit helleren ("highlights") oder dunkleren ("lowlights") Partien (Strähnchen) erzeugt werden.

Es hat sich gezeigt, dass eine Vorbehandlung bei leicht erhöhten Temperaturen die multitonalen Effekte noch lebendiger wirken lässt. Erfindungsgemäß bevorzugte Verfahren sind dadurch gekennzeichnet, dass das kosmetische Mittel (M1) in Verfahrensschritt b) bei einer Temperatur von 20 °C bis 120 °C, insbesondere von 30 °C bis 120 °C, Einwirken gelassen wird. Temperaturen von 30 °C bis 120 °C, bevorzugt von 40 °C bis 120 °C können beispielsweise unter Verwendung eines Heißluftföns oder einer Trockenhaube erreicht werden.

Um multitonale Färbungen zu erzeugen, wird das kosmetische Mittel (M1) nicht gleichmäßig auf die keratinischen Fasern appliziert. Vielmehr wird das kosmetische Mittel (M1) nur auf einzelne Strähnchen appliziert.

In diesem Zusammenhang ist es besonders bevorzugt, wenn das kosmetische Mittel (M1) in Verfahrensschritt a) nur auf einzelne Strähnchen appliziert wird. Unter dem Begriff "Strähnchen" wird erfindungsgemäß ein von der Gesamtheit an keratinischen Fasern abgetrennter Teil verstanden, welcher aus mindestens 2, bevorzugt mindestens 50, insbesondere mindestens 100, keratinischen Fasern besteht.

Im Anschluss an die Einwirkzeit des Vorbehandlungsmittels werden die keratinischen Fasern nicht ausgespült oder abfrottiert. Vielmehr wird in Verfahrensschritt c) des erfindungsgemäßen Verfahrens ein kosmetisches Mittel (M2) auf die noch mit dem kosmetischen Mittel (M1) beaufschlagten keratinischen Fasern appliziert. Die durch Applikation des kosmetischen Mittels (M2) auf den keratinischen Fasern entstandene Mischung aus den kosmetischen Mitteln (M1) und (M2) wird in Verfahrensschritt d) des erfindungsgemäßen Verfahrens für einen Zeitraum von 1 bis 70 Minuten Einwirken gelassen.

Erfindungsgemäß sind jedoch eher kürzere Einwirkzeiten der kosmetischen Mittel (M1) und (M2) in Verfahrensschritt d) bevorzugt. Besonders bevorzugte erfindungsgemäße Verfahren sind dadurch gekennzeichnet, dass die kosmetischen Mittel (M1) und (M2) in Verfahrensschritt d) für einen Zeitraum
von 1 bis 60 Minuten, vorzugsweise von 5 bis 50 Minuten, insbesondere von 10 bis 45 Minuten, Einwirken gelassen werden.

Da das kosmetische Mittel (M1) in Verfahrensschritt b) des erfindungsgemäßen Verfahrens bereits eine gewisse Zeit auf den keratinischen Fasern belassen wurde, haben diese keratinischen Fasern zu den Inhaltsstoffen des kosmetischen Mittels (M1) längeren Kontakt als zu denen des kosmetischen Mittels (M2). Wenn das kosmetische Mittel (M1) nur auf einzelne Strähnchen bzw. in einzelnen Bereichen abgewendet wurde, konnten die Inhaltsstoffe des kosmetischen Mittels (M1) in diesen Bereichen intensiver einwirken und somit die Wirkung der Inhaltsstoffe des kosmetischen Mittels (M2) in diesen Bereichen verstärken oder abschwächen, wodurch eine dunklere oder hellere Färbung dieser Bereiche erzielt wird.

Nach dem Ausspülen der kosmetischen Mittel (M1) und (M2) in Verfahrensschritt e) des erfindungsgemäßen Verfahrens wird, ohne einen weiteren Schritt durchführen zu müssen, unmittelbar ein multitonales Farbergebnis erhalten.

Bei dem kosmetischen Mittel (M1) bzw. dem Vorbehandlungsmittel handelt es sich um ein Haarfärbemittel, welches mindestens einen direktziehenden Farbstoff (M1-1) enthält. Bei direktziehenden Farbstoffen handelt sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Gemäß einer bevorzugten Ausführungsform des Erfindungsgegenstands ist der mindestens eine direktziehende Farbstoff (M1-1) ausgewählt aus der Gruppe von anionischen direktziehenden Farbstoffen, kationischen direktziehenden Farbstoffen, nichtionischen direktziehenden Farbstoffen sowie deren Mischungen.

In diesem Zusammenhang kann es erfindungsgemäß vorgesehen sein, dass der anionische direktziehende Farbstoff ausgewählt ist aus der Gruppe von Acid Yellow 1, Acid Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Tetrabromphenolblau und/oder deren physiologisch verträglichen Salzen.

Im Rahmen dieser Ausführungsform kann es weiterhin vorgesehen sein, dass der kationische direktziehende Farbstoff ausgewählt ist aus der Gruppe von Basic Blue 7, Basic Blue 26, Basic Violet 2 und Basic Violet 14, Basic Yellow 57, Basic Red 76, Basic Blue 99, Basic Brown 16 und Basic Brown 17 und HC Blue 16, sowie Basic Yellow 87, Basic Orange 31 und Basic Red 51 und/oder deren physiologisch verträglichen Salzen.

Darüber hinaus kann es im Rahmen dieser Ausführungsform auch vorgesehen sein, dass der nichtionische direktziehende Farbstoff ausgewählt ist aus der Gruppe von HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure, 2-Amino-6-chloro-4-nitrophenol, 4-Ethylamino-3-nitrobenzoesäure und 2-Chlor-6-ethylamino-4-nitrophenol und/oder deren physiologisch verträglichen Salzen, bevorzugt 2-Amino-6-chloro-4-nitrophenol und/oder 4-Amino-3-nitrophenol und/oder deren physiologisch verträglichen Salzen.

Im Rahmen des erfindungsgemäßen Verfahrens besonders bevorzugt eingesetzte Vorbehandlungsmittel (M1) enthalten mindestens eine direktziehende Farbstoff (M1-1), welcher ausgewählt ist aus der Gruppe von 2-Amino-6-chloro-4-nitrophenol, HC Blue 12, HC Yellow 2, HC Violet 14D und/oder deren physiologisch verträglichen Salzen sowie deren Mischungen. Bei Einsatz dieser speziellen direktziehenden Farbstoffe wird eine besonders ausgewogene und subtile Nuancenbildung während des erfindungsgemäßen Verfahrens bzw. bei der multitonalen Färbung erreicht.

Erfindungsgemäß bevorzugt enthält das kosmetische Mittel (M1) den mindestens einen direktziehenden Farbstoff (M1-3) in einer Gesamtmenge von 0,00001 bis 5,0 Gew.-%, vorzugsweise von 0,0001 bis 5,5 Gew.-%, vorzugsweise von 0,0005 bis 5,0 Gew.-%, bevorzugt von 0,001 bis 4,5 Gew.-%, weiter bevorzugt von 0,001 bis 4,0 Gew.-%, insbesondere von 0,001 bis 3,0 Gew.-%, bezogen auf das Gesamtgewicht des kosmetischen Mittels (M1). Die zuvor genannten Mengen der direktziehenden Farbstoffe führen zu besonders ausgewogenen Nuancen im Rahmen der multitonalen Färbung gemäß dem erfindungsgemäßen Verfahren.

Um die gewünschte Viskosität einzustellen, und ein Herablaufen des kosmetischen Mittels (M1) während der Auftragung auf die keratinischen Fasern sowie während der Einwirkzeit in Verfahrensschritt b) zu vermeiden, enthalten die kosmetischen Mittel (M1) mindestens ein Verdickungsmittel (M1-2).

Gemäß einer bevorzugten Ausführungsform des Erfindungsgegenstands ist das mindestens eine Verdickungsmittel (M1-2) ausgewählt aus der Gruppe von verdickenden Polysacchariden, verdickenden synthetischen Polymeren, verdickenden anorganischen Verbindungen sowie deren Mischungen.

In diesem Zusammenhang kann es erfindungsgemäß vorgesehen sein, dass das verdickende Polysaccharid ausgewählt ist aus der Gruppe von Xanthan, Cellulosen, Cellulosederivaten, Curdlan, Alginen, Alginaten, Glucanen, Pullulanen, Amylosen, Traganth, Karaya-Gummi, Ghatti-Gummi, Agar, Carrageenan, Chitin, Chitosan, Gummi arabicum, Gellan, Guar Gum, Johannisbrotkernmehl sowie deren Mischungen, bevorzugt Xanthan, Cellulosen, Cellulosederivaten sowie deren Mischungen.

Im Rahmen dieser Ausführungsform kann es weiterhin vorgesehen sein, dass das verdickende synthetische Polymer ausgewählt ist aus der Gruppe von vernetzten Homo- oder Copolymeren der Acrylsäure, der Methacrylsäure sowie deren Salzen und Alkylestern, Homo- oder Copolymere von Acrylsäureamiden und/oder Methacrylsäureamiden, Copolymere aus Acrylsäure und Acrylsäureamiden sowie deren Mischungen, bevorzugt vernetzten Homo- oder Copolymeren der Acylsäure, der Methacrylsäure sowie deren Salzen und Alkylestern, vernetzten Copolymeren der ethoxylierten Alkylester der Methacrylsäure und der sulfonierten Acrylsäureamide sowie deren Salzen und vernetzten Copolymeren der Methacrylsäure, der Acrylsäureamide und der sulfonierten Acrylsäureamide sowie deren Salzen. Besonders bevorzugt ist das unter der INCI-Bezeichnung bekannte vernetzte Copolymer Ammonium Acryloyldimethyltaurate / Beheneth-25 methacrylate Crosspolymer, welches beispielsweise unter dem Handelsnamen Aristoflex HMB der Firma Clariant kommerziell erhältlich ist. Im Rahmen dieser Ausführungsform ebenfalls besonders bevorzugt ist das vernetzte Copolymer mit der INCI-Bezeichnung Acrylates/C10-30 Alkyl Acrylate Crosspolymer, welches unter den Handelsnamen Carbopol der Firma Lubrizol erhältlich ist. Darüber hinaus ist im Rahmen dieser Ausführungsform das unter der INCI-Bezeichnung Polyacrylate Crosspolymer-11 vernetzte Copolymer bevorzugt, welches unter dem Handelsnamen Aristoflex Velvet von der Firma Clariant kommerziell erhältlich ist.

Darüber hinaus kann es im Rahmen dieser Ausführungsform auch vorgesehen sein, dass die verdickende anionische Verbindung ausgewählt ist aus der Gruppe von Elektrolyten, insbesondere Natriumchlorid und Kaliumchlorid, Schichtsilicaten, Magnesium-Aluminium-Silicate, gegebenenfalls modifizierte Bentonite, insbesondere gegebenenfalls modifizierte Smektite sowie deren Mischungen.

Im Rahmen des erfindungsgemäßen Verfahrens besonders bevorzugt eingesetzte kosmetische Mittel (M1) enthalten mindestens eine Verdickungsmittel (M1-2) ausgewählt ist aus der Gruppe von Cellulose, Cellulosederivaten, Xanthan, vernetzten Homo- oder Copolymeren der Acrylsäure, der Methacrylsäure und deren Salzen, vernetzten Copolymeren der ethoxylierten Alkylester der Methacrylsäure und der sulfonierten Acrylsäureamide sowie deren Salzen, vernetzten Copolymeren der Methacrylsäure, der Acrylsäureamide und der sulfonierten Acrylsäureamide sowie deren Salzen und Mischungen dieser Verdickungsmittel und Mischungen dieser Verdickungsmittel. Um das Vorpenetrationsmittel (M1) sauber und lokal begrenzt applizieren zu können, hat sich eine gelartige Viskosität des Mittels als vorteilhaft erwiesen. Die gelartigen Vorbehandlungsmittel (M1) gewährleisten zum einen eine gute und gleichmäßige Verteilbarkeit auf den keratinischen Fasern und führen nicht zu einem Verlaufen oder Ausfließen während der Einwirkzeit in Verfahrensschritt b). Auf diese Weise ist die Applikation und Einwirkung des Vorbehandlungsmittels (M1) auf begrenzte Strähnchen bzw. Bereiche möglich, so dass ein hervorragendes multitonales Färbeergebnis ohne Verwischen der Strähnchen infolge eines Verlaufens des Vorbehandlungsmittels (M1) resultiert.

Im Rahmen des erfindungsgemäßen Verfahrens kann es zudem besonders bevorzugt sein, wenn in den kosmetischen Mitteln (M1) zwei voneinander verschiedene Verdickungsmittel (M1-2), ausgewählt aus vernetzten Homo- oder Copolymeren der Acrylsäure, der Methacrylsäure und deren Salzen sowie Xanthan, eingesetzt werden.

Erfindungsgemäß bevorzugt enthält das kosmetische Mittel (M1) das mindestens eine Verdickungsmittel (M1-2) in einer Gesamtmenge von 0,1 bis 5,0 Gew.-%, vorzugsweise von 0,3 bis 4,5 Gew.-%, bevorzugt von 0,5 bis 4,0 Gew.-%, weiter bevorzugt von 0,7 bis 3,5 Gew.-%, insbesondere von 0,8 bis 3,0 Gew.-%, bezogen auf das Gesamtgewicht des kosmetischen Mittels (M1). Die zuvor genannten Mengen der Verdickungsmittel gewährleisten eine ausreichende Verdickung, so dass ein Verlaufen des Vorbehandlungsmittels (M1) während der Einwirkzeit in Verfahrensschritt b) und ein hierdurch verursachtes Verwischen des multitonalen Färbeergebnisses verhindert wird. Weiterhin stellen diese Mengen an Verdickungsmittel eine gute und gleichmäßige Verteilbarkeit der Vorbehandlungsmittel (M1) auf den keratinischen Fasern sicher.

Erfindungsgemäß ist das kosmetische Mittel (M1) frei von Oxidationsfarbstoffvorprodukten (M1-3). Unter "Oxidationsfarbstoffvorprodukten" werden im Rahmen der vorliegenden Erfindung Verbindungen vom Entwicklertyp und vom Kupplertyp verstanden, welche durch Einwirkung eines Oxidationsmittels, beispielsweise Wasserstoffperoxid, in oxidativen Kupplungsreaktionen die entsprechenden Farbstoffe ausbilden.

Der Begriff "frei von" bedeutet hierbei im Rahmen der vorliegenden Erfindung, dass keine Oxidationsfarbstoffvorprodukte in den kosmetischen Mitteln (M1) enthalten sind. "Frei von" bedeutet daher konkreter, dass die in Verfahrensschritt a) eingesetzten kosmetischen Mittel (M1) weniger als 1,0 Gew.-%, vorzugsweise weniger als 0,5 Gew.-%, bevorzugt weniger als 0,25 Gew.-%, weiter bevorzugt weniger als 0,1 Gew.-%, insbesondere 0,0 Gew.-%, bezogen auf das Gesamtgewicht des kosmetischen Mittels (M1), Oxidationsfarbstoffvorprodukte enthalten.

Erfindungsgemäß bevorzugte Verfahren sind daher dadurch gekennzeichnet, dass das in Verfahrensschritt a) eingesetzte kosmetische Mittel (M1) weniger als 1,0 Gew.-%, vorzugsweise weniger als 0,5 Gew.-%, bevorzugt weniger als 0,25 Gew.-%, weiter bevorzugt weniger als 0,1 Gew.-%, insbesondere 0,0 Gew.-%, bezogen auf das Gesamtgewicht des kosmetischen Mittels (M1), Oxidationsfarbstoffvorprodukte, insbesondere Entwickler- und Kupplerkomponenten, enthält.

Im Rahmen des erfindungsgemäßen Verfahrens ist es zur Vermeidung eines Verlaufens des Vorbehandlungsmittels (M1) und somit zur Vermeidung eines Verwischens des multitonalen Färbeergebnisses von Vorteil, wenn die Vorbehandlungsmittel (M1) in Form von gelartigen kosmetischen Mitteln vorliegen. Erfindungsgemäß verwendete kosmetische Mittel (M1) weisen daher eine dynamische Viskosität von 10.000 bis 50.000 mPa*s, jeweils gemessen mit Brookfield RDV II+, Spindel Nr. 4, 4 rpm, 20 °C, auf.

Zusätzlich zu dem bzw. den direktziehenden Farbstoff(en) sowie dem bzw. den Verdickungsmittel(n) kann das im erfindungsgemäßen Verfahren eingesetzte kosmetische Mittel (M1) weitere Inhaltsstoffe enthalten.

Erfindungsgemäß bevorzugt enthält das kosmetische Mittel (M1) zusätzlich mindestens eine weitere Verbindung, ausgewählt aus der Gruppe von (i) Tensiden; (ii) Glykolen; (iii) Alkalisierungsmitteln; sowie (iv) deren Mischungen.

Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden und Emulgatoren ist die orientierte Absorption an Grenzflächen sowie die Aggregation zu Mizellen und die Ausbildung von lyotrophen Phasen.

Im Rahmen der vorliegenden Erfindung einsetzbare Tenside sind ausgewählt aus der Gruppe von nichtionischen Tensiden, anionischen Tensiden, amphoteren Tensiden, zwitterionischen Tensiden, kationischen Tensiden sowie deren Mischungen.

Erfindungsgemäß besonderes bevorzugt werden in dem erfindungsgemäßen Verfahren kosmetische Mittel (M1) eingesetzt, welche zusätzlich mindestens ein nichtionisches Tensid aus der Gruppe von (i) Alkylenoxid-Anlagerungsprodukten an Alkohole mit 8 bis 30 Kohlenstoffatomen oder Carbonsäuren mit 8 bis 30 Kohlenstoffatomen, welche jeweils 2 bis 30 Mol Ethylenoxid pro Mol Alkohol oder Carbonsäure enthalten; (ii) Carbonsäureestern von ethoxyliertem und/oder propoxyliertem Glycerin mit 8 bis 30 Kohlenstoffatomen in der Carbonsäurekette und 1 bis 30 Mol Ethylenoxid und/oder Propylenoxid pro Mol Glycerin; sowie (iii) deren Mischungen enthalten.

Besonders bevorzugte erfindungsgemäße Verfahren sind daher dadurch gekennzeichnet, dass die kosmetischen Mittel (M1) zusätzlich mindestens ein nichtionisches Tensid aus der Gruppe von Carbonsäureestern von ethoxyliertem und/oder propoxyliertem Glycerin mit 8 bis 30 Kohlenstoffatomen in der Carbonsäurekette und 1 bis 30 Mol Ethylenoxid und/oder Propylenoxid pro Mol Glycerin in einer Gesamtmenge von 0,1 bis 10 Gew.%, vorzugsweise von 0,5 bis 5 Gew.%, insbesondere von 0,8 bis 3 Gew.%, bezogen auf das Gesamtgewicht des kosmetischen Mittels (M1), enthalten.

Weiterhin können die Vorbehandlungsmittel (M1) zusätzlich mindestens ein anionisches Tensid enthalten. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in einer Gesamtmenge von 0,1 bis 45 Gew.-%, vorzugsweise von 1 bis 30 Gew.-%, insbesondere von 1 bis 15 Gew.-%, bezogen auf das Gesamtgewicht des kosmetischen Mittels (M1), eingesetzt.

Darüber hinaus ist es ebenfalls möglich, dass die Vorbehandlungsmittel (M1) zusätzlich mindestens ein zwitterionisches und/oder amphoteres Tensid enthalten. Bevorzugte zwitterionische Tenside sind Betaine, N-Alkyl-N,N-dimethylammonium-glycinate, N-Acyl-aminopropyl-N,N-di-methylammoniumglycinate, und 2-Alkyl-3-carboxymethyl-3-hydroxyethyl-imidazoline. Ein besonders bevorzugtes zwitterionisches Tensid ist unter der INCI-Bezeichnung Cocamidopropyl Betaine bekannt. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, Kokosacylaminoethylaminopropionat und C₁₂-C₁₈-Acylsarcosin. Die zwitterionischen und/oder amphoteren Tenside werden einer Gesamtmenge von 0,1 bis 45 Gew.-%, vorzugsweise von 1 bis 30 Gew.-%, insbesondere von 1 bis 15 Gew.-%, bezogen auf das Gesamtgewicht des kosmetischen Mittels (M1), eingesetzt.

Die Vorbehandlungsmittel (M1) können zusätzlich mindestens eine Verbindung aus der Gruppe der Glykole enthalten. Unter dem Begriff "Glykole" werden erfindungsgemäß Verbindungen verstanden, welche 2 Hydroxylgruppen aufweisen.

Erfindungsgemäß geeignete Glykole sind ausgewählt aus der Gruppe von Ethylenglykol, Propylenglykol (1,2-Propandiol), Ethylenglykolmonomethylether, Trimethylenglykol, Triethylenglykol, Polyethylenglykol, Neopentylglykol sowie deren Mischungen.

Besonders bevorzugte erfindungsgemäße Verfahren sind dadurch gekennzeichnet, dass die kosmetischen Mittel (M1) zusätzlich mindestens ein Glykol aus der Gruppe von Ethylenglykol, Propylenglykol (1,2-Propandiol), Polyethylengykol sowie deren Mischungen in einer Gesamtmenge von 0,1 bis 10 Gew.%, vorzugsweise von 0,5 bis 5 Gew.%, insbesondere von 0,8 bis 3 Gew.%, bezogen auf das Gesamtgewicht des kosmetischen Mittels (M1), enthalten.

Die im Rahmen des erfindungsgemäßen Verfahrens verwendeten Vorbehandlungsmittel (M1) können ebenfalls mindestens ein Alkalisierungsmittel enthalten.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, welcher mindestens eine Hydroxylgruppe trägt. Erfindungsgemäß ganz besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol sowie deren Mischungen. Ein insbesondere bevorzugtes Alkanolamin ist Monoethanolamin. Geeignete basische Aminsäuren sind Lysin, Arginin und Ornithin. Erfindungsgemäße, anorganische Alkalisierungsmittel sind bevorzugt ausgewählt aus der Gruppe von Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat.

Erfindungsgemäß besonders bevorzugte Verfahren sind dadurch gekennzeichnet, dass das kosmetische Mittel (M1) ein oder mehrere Alkalisierungsmittel aus der Gruppe von Natriumhydroxid, Kaliumhydroxid, Ammoniak, Monoethanolamin und 2-Amino-2-methylpropanol in einer Gesamtmenge von 0,5 bis 8,0 Gew.-%, vorzugsweise von 0,1 bis 6,0 Gew.-%, insbesondere von 0,5 bis 5,0 Gew.-%, bezogen auf das Gesamtgewicht des kosmetischen Mittels (M1), enthält.

Die im Rahmen des erfindungsgemäßen Verfahrens verwendeten Vorbehandlungsmittel (M1) weisen in der Regel einen basischen pH-Wert, insbesondere zwischen pH 7,0 und pH 14, auf. Diese pH-Werte sind erforderlich, um eine Öffnung der äußeren Schuppenschicht (Cuticula) zu gewährleisten und eine Penetration der Oxidationsfarbstoffvorprodukte in das Haar zu ermöglichen.

Erfindungsgemäße Verfahren sind dadurch gekennzeichnet, dass das kosmetische Mittel (M1) einen pH-Wert von pH 9,2 bis pH 10,5, aufweist. Die Einstellung dieser pH-Werte kann bevorzugt unter Verwendung der zuvor angeführten Alkalisierungsmittel erfolgen.

Um das natürliche und multitonale Färbeergebnis am Ende des erfindungsgemäßen Verfahrens besonders deutlich und überraschend hervortreten zu lassen, ist das Vorbehandlungsmittel (M1) für sich alleine vorzugsweise nicht in der Lage, als separates Bleich- oder Aufhellmittel eingesetzt zu werden. Hierzu ist es von besonderem Vorteil, wenn die kosmetischen Mittel (M1) frei sind von Oxidationsmitteln, insbesondere frei sind von Wasserstoffperoxid und/oder Persulfaten.

Der Begriff "frei von" bedeutet hierbei im Rahmen der vorliegenden Erfindung, dass keine intentionell zugesetzten Oxidationsmittel in den kosmetischen Mitteln (M1) enthalten sind. Gleichwohl können Spuren dieser Oxidationsmittel als Verunreinigung oder Begleitstoff über andere Rohstoffe in die kosmetischen Mittel (M1) eingebracht werden. "Frei von" bedeutet daher konkreter, dass die kosmetischen Mittel (M1) weniger als 1 Gew.-%, vorzugsweise weniger als 0,5 Gew.-%, bevorzugt weniger als 0,25 Gew.-%, weiter bevorzugt weniger als 0,1 Gew.-%, insbesondere weniger als 0,01 Gew.-%, bezogen auf das Gesamtgewicht des kosmetischen Mittels (M1), Oxidationsmittel enthalten. Erfindungsgemäß bevorzugte Verfahren sind daher dadurch gekennzeichnet, dass das in Verfahrensschritt a) eingesetzte kosmetische Mittel (M1) weniger als 1 Gew.-%, vorzugsweise weniger als 0,5 Gew.-%, bevorzugt weniger als 0,25 Gew.-%, weiter bevorzugt weniger als 0,1 Gew.-%, insbesondere weniger als 0,01 Gew.-%, bezogen auf das Gesamtgewicht des kosmetischen Mittels (M1), Peroxoverbindungen enthält. Unter Peroxoverbindungen werden im Rahmen der vorliegenden Erfindung Verbindungen verstanden, welche mindestens ein Peroxidanion O₂²⁻ beziehungsweise mindestens eine Peroxygruppe -O-O- enthalten.

Erfindungsgemäß besonders bevorzugte Verfahren sind dadurch gekennzeichnet, dass das in Verfahrensschritt a) eingesetzte kosmetische Mittel (M1) weniger als 1 Gew.-%, vorzugsweise weniger als 0,5 Gew.-%, bevorzugt weniger als 0,25 Gew.-%, weiter bevorzugt weniger als 0,1 Gew.-%, insbesondere weniger als 0,01 Gew.-%, bezogen auf das Gesamtgewicht des kosmetischen Mittels (M1), Wasserstoffperoxid enthält.

In Verfahrensschritt c) des erfindungsgemäßen Verfahrens wird ein kosmetisches Mittel (M2) auf die keratinischen Fasern appliziert, welche noch mit dem Mittel (M1) beaufschlagt sind. Dieses kosmetische Mittel (M2), welches im nachfolgenden auch als Färbemittel bezeichnet wird, enthält mindestens ein Oxidationsfarbstoffvorprodukt (M2-1) und mindestens ein Oxidationsmittel (M2-2).

Bevorzugte kosmetische Mittel (M2) enthalten mindestens ein Oxidationsfarbstoffvorprodukt vom Entwickler- und/oder Kupplertyp. Entsprechende erfindungsgemäße Verfahren, bei welchen das kosmetische Mittel (M2) als Oxidationsfarbstoffvorprodukt (M2-1) ein oder mehrere Oxidationsfarbstoffvorprodukte vom Entwicklertyp enthält, sind erfindungsgemäß bevorzugt.

Es hat sich gezeigt, dass sich der Einsatz von bestimmten Oxidationsfarbstoffvorprodukten (M2-1) vom Entwicklertyp in bestimmten Mengen in den Färbemitteln (M2) gut eignet, um besonders lebendige, wasch-, reib-, schweiß- und UV-echte multitonalen Färbungen zu erzeugen.

Erfindungsgemäß bevorzugt ist daher das mindestens eine Oxidationsfarbstoffvorprodukt (M2-1) vom Entwicklertyp ausgewählt aus der Gruppe von 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on, p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol sowie deren physiologisch verträglichen Salzen und deren Mischungen.

Um natürliche Färbungen zu erhalten, müssen üblicherweise mehrere Oxidationsfarbstoffvorprodukte (M2-1) vom Entwicklertyp eingesetzt werden. Bevorzugte kosmetische Mittel (M2) sind daher dadurch gekennzeichnet, dass das mindestens eine Oxidationsfarbstoffvorprodukt (M2-1) vom Entwicklertyp ausgewählt ist aus mindestens einer der folgenden Kombinationen: p-Toluylendiamin/2-(2-Hydroxyethyl)-p-phenylendiamin; p-Toluylendiamin/2-Methoxymethyl-p-phenylendiamin; p-Toluylendiamin/N,N-Bis-(2-hydroxyethyl)-p-phenylendiamin; p-Toluylendiamin/2-Methoxymethyl-p-phenylendiamin; p-Toluylendiamin/N-(4-Amino-3-methylphenyl)-N-[3-(1 H-imidazol-1-yl)propyl]amin; p-Toluylendiamin/Bis-(2-hydroxy-5-aminophenyl)methan; p-Toluylendiamin/4-Amino-3-methylphenol; p-Toluylendiamin/4,5-Diamino-1-(2-hydroxyethyl)pyrazol; p-Toluylendiamin/2,4,5,6-Tetraaminopyrimidin; 2-(2-Hydroxyethyl)-p-phenylendiamin /2-Methoxymethyl-p-phenylendiamin; 2-(2-Hydroxyethyl)-p-phenylendiamin /N,N-Bis-(2-hydroxyethyl)-p-phenylendiamin; 2-(2-Hydroxyethyl)-p-phenylendiamin /2-Methoxymethyl-p-phenylendiamin; 2-(2-Hydroxyethyl)-p-phenylendiamin /N-(4-Amino-3-methylphenyl)-N-[3-(1H-imidazol-1-yl)propyl]amin; 2-(2-Hydroxyethyl)-p-phenylendiamin /Bis-(2-hydroxy-5-aminophenyl)methan; 2-(2-Hydroxyethyl)-p-phenylendiamin/4-Amino-3-methylphenol; 2-(2-Hydroxyethyl)-p-phenylendiamin /4,5-Diamino-1-(2-hydroxyethyl)pyrazol; 2-(2-Hydroxyethyl)-p-phenylendiamin /2,4,5,6-Tetraaminopyrimidin; 2-Methoxymethyl-p-phenylendiamin/2-(2-Hydroxyethyl)-p-phenylendiamin; 2-Methoxymethyl-p-phenylendiamin /2-Methoxymethyl-p-phenylendiamin; 2-Methoxymethyl-p-phenylendiamin /N,N-Bis-(2-hydroxyethyl)-p-phenylendiamin; 2-Methoxymethyl-p-phenylendiamin /2-Methoxymethyl-p-phenylendiamin; 2-Methoxymethyl-p-phenylendiamin /N-(4-Amino-3-methylphenyl)-N-[3-(1H-imidazol-1-yl)-propyl]amin; /Bis-(2-hydroxy-5-aminophenyl)methan; 2-Methoxymethyl-p-phenylendiamin/4-Amino-3-methylphenol; 2-Methoxymethyl-p-phenylendiamin /4,5-Diamino-1-(2-hydroxyethyl)pyrazol; 2-Methoxymethyl-p-phenylendiamin /2,4,5,6-Tetraaminopyrimidin und/oder 4-und Amino-3-methylphenol/4,5-Diamino-1-(2-hydroxyethyl)pyrazol und/oder deren physiologisch verträgliche Salze.

Gemäß einer besonders bevorzugten Ausführungsform des ersten Erfindungsgegenstands ist das mindestens eine Oxidationsfarbstoffvorprodukt (M2-1) vom Entwicklertyp ausgewählt aus der Gruppe von p-Toluylendiamin, 4-Amino-3-methylphenol, 4,5-Diamino-1-(2-hydroxyethyl)pyrazol, 2,4,5,6-Tetraaminopyrimidin und/oder deren physiologisch verträglichen Salzen sowie deren Mischungen und ist in einer Gesamtmenge von 0,0005 bis 3,0 Gew.-%, vorzugsweise von 0,001 bis 2,75 Gew.-%, bevorzugt 0,0025 bis 2,5 Gew.-%, insbesondere 0,005 bis 2,0 Gew.-%, bezogen auf das Gesamtgewicht des kosmetischen Mittels (M2), enthalten. Es hat sich gezeigt, dass der Einsatz dieser speziellen Oxidationsfarbstoffvorprodukte (M2-1) vom Entwicklertyp in den zuvor angeführte Mengen in den Färbemitteln (M2) zu besonders lebendigen, wasch-, reib-, schweiß- und UV-echten multitonalen Färbungen führt.

Das Färbemittel (M2) kann als weiteren Bestandteil mindestens ein Oxidationsfarbstoffvorprodukt (M2-1) vom Kupplertyp enthalten. Oxidationsfarbstoffvorprodukte vom Kupplertyp bilden im Rahmen der oxidativen Färbung allein keine signifikante Färbung aus, sondern benötigen für eine ausreichende Färbung die Gegenwart von Oxidationsfarbstoffvorprodukten vom Entwicklertyp. Oxidationsfarbstoffvorprodukte vom Kupplertyp im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus.

Im Rahmen der vorliegenden Erfindung ist es bevorzugt, wenn das mindestens eine Oxidationsfarbstoffvorprodukt (M2-1) vom Kupplertyp ausgewählt ist aus der Gruppe von m-Aminophenol und dessen Derivaten, o-Aminophenol und dessen Derivaten, m-Diaminobenzol und dessen Derivaten, o-Diaminobenzol und dessen Derivaten, Di- und Trihydroxybenzolderivaten, Pyridinderivaten, Naphthalinderivaten, Morpholinderivaten, Chinoxalinderivaten, Pyrazolderivaten, Indolderivaten, Pyrimidinderivaten, Methylendioxybenzolderivaten und/oder deren physiologisch verträglichen Salzen sowie deren Mischungen.

Bevorzugte erfindungsgemäße Verfahren sind dadurch gekennzeichnet, dass das mindestens eine Oxidationsfarbstoffvorprodukt (M2-1) vom Kupplertyp ausgewählt ist aus der Gruppe von Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin, 1-Naphthol und/oder deren physiologisch verträglichen Salzen sowie deren Mischungen und in einer Gesamtmenge von 0,001 bis 2,0 Gew.-%, vorzugsweise von 0,0025 bis 1,75 Gew.-%, bevorzugt 0,0025 bis 1,5 Gew.-%, insbesondere 0,005 bis 1,25 Gew.-%, bezogen auf das Gesamtgewicht des kosmetischen Mittels (M2), enthalten ist. Die vorstehend genannten Kupplerkomponenten in den angegebenen Mengenbereichen führen in Kombination mit den zuvor angeführten Entwicklerkomponenten in dem Färbemittel (M2) zu besonders intensiven und beständigen multitonalen Färbeergebnissen.

Besonders ansprechend aussehende multitonale Färbungen werden erhalten, wenn das kosmetische Mittel (M2) das mindestens eine Oxidationsfarbstoffvorprodukt (M2-1) in einer Gesamtmenge von 0,01 bis 4,0 Gew.-%, vorzugsweise von 0,008 bis 3,75 Gew.-%, bevorzugt 0,005 bis 3,5 Gew.-%, insbesondere 0,005 bis 3,2 Gew.-%, bezogen auf das Gesamtgewicht des kosmetischen Mittels (M2), enthält. Die vorstehend genannten Mengen des mindestens einen Oxidationsfarbstoffvorprodukts (M2-1) führen zu multitonalen Färbungen, welche besonders intensive und leuchtende Farben sowie eine hohe Beständigkeit gegen Umwelteinflüsse, wie Haarwäschen, UV-Licht, Schweiß und Reibung, aufweisen.

Das Färbemittel (M2) kann zusätzlich direktziehende Farbstoffe enthalten, um eine ausgewogene Nuancierung der multitonalen Färbungen zu gewährleisten. Gemäß einer bevorzugten Ausführungsform des ersten Erfindungsgegenstands enthält das kosmetische Mittel (M2) zusätzlich mindestens einen direktziehenden Farbstoff aus der Gruppe von anionischen direktziehenden Farbstoffen, kationischen direktziehenden Farbstoffen, nichtionischen direktziehenden Farbstoffen sowie deren Mischungen.

Geeignete und bevorzugte direktziehende Farbstoffe wurden bereits im Zusammenhang mit dem Vorbehandlungsmittel (M1) detailliert beschrieben. Die entsprechenden Verbindungen können auch in den Färbemitteln (M2) eingesetzt werden. Es hat sich jedoch gezeigt, dass sich der Einsatz von bestimmten direktziehenden Farbstoffen in bestimmten Mengen in den Färbemitteln (M2) besonders gut eignet, da in diesem Fall eine besonders ausgewogene Nuancierung der multitonalen Färbungen gewährleistet werden kann.

Im Rahmen einer Ausführungsform des erfindungsgemäßen Verfahrens besonders bevorzugt eingesetzte kosmetische Mittel (M2) enthalten daher zusätzlich mindestens einen direktziehenden Farbstoff, welcher ausgewählt ist aus der Gruppe von 2-Amino-6-chloro-4-nitrophenol, HC Blue 12, HC Yellow 2, HC Violet 14D und/oder deren physiologisch verträglichen Salzen sowie deren Mischungen.

Erfindungsgemäße Verfahren im Rahmen dieser Ausführungsform sind dadurch gekennzeichnet, dass das kosmetische Mittel (M2) den mindestens einen direktziehenden Farbstoff in einer Gesamtmenge von 0,00005 bis 5,0 Gew.-%, vorzugsweise von 0,0001 bis 4,0 Gew.-%, bevorzugt von 0,0005 bis 3,0 Gew.-%, weiter bevorzugt von 0,0001 bis 2,0 Gew.-%, insbesondere von 0,0005 bis 1,5 Gew.-%, bezogen auf das Gesamtgewicht des kosmetischen Mittels (M2).

Wenn ein direktziehende Farbstoff in dem Färbemittel (M2) enthalten ist, dann ist es im Rahmen der vorliegenden Erfindung bevorzugt, wenn das Vorbehandlungsmittel (M1) eine größere Gesamtmenge an direktziehenden Farbstoffen (M1-1) enthält als das kosmetische Mittel (M2). Dies führt zu besonders intensiven und lebendigen multitonalen Färbungen, welche darüber hinaus eine hohe Beständigkeit gegen Umwelteinflüsse, wie beispielsweise Haarwäschen, Schweiß, UV-Licht oder Reibung, aufweisen.

Es ist daher in diesem Zusammenhang bevorzugt, wenn das Mengenverhältnis der Gesamtmenge aller direktziehender Farbstoffe (M1-1) in dem kosmetischen Mittel (M1) zur Gesamtmenge aller direktziehender Farbstoffe in dem kosmetischen Mittel (M2) einen Wert von 1:5 bis 1:1, vorzugsweise von 150:1 bis 250:1, bevorzugt von 450:1 bis 550:1, weiter bevorzugt von 900 :1 bis 1.100:1, noch weiter bevorzugt von 2.500 :1 bis 3.500:1, insbesondere von 5.500:1 bis 6.500:1, aufweist.

Weiterhin kann in diesem Zusammenhang eine Variation der Nuancierung der multitonalen Färbung durch entsprechende Wahl der in den kosmetischen Mitteln (M1) und (M2) eingesetzten direktziehenden Farbstoffe erreicht werden. Für eine sehr natürlich wirkende multitonale Färbung mit weichen Übergängen sind daher erfindungsgemäße Verfahren bevorzugt, bei welchen die kosmetischen Mittel (M1) und (M2) identische direktziehende Farbstoffe enthalten.

Werden hingegen stärkere Kontraste gewünscht, welche sich in einem brillanteren multitonalen Erscheinungsbild der Färbung äußern, haben sich erfindungsgemäße Verfahren bewährt, bei welchen die kosmetischen Mittel (M1) und (M2) unterschiedliche direktziehende Farbstoffe enthalten.

Die Färbemittel (M2) können weiterhin zusätzliche Wirk-, Hilfs- und Zusatzstoffe enthalten, um die Färbeleistung zu verbessern und weitere gewünschte Eigenschaften der kosmetischen Mittel (M2) einzustellen.

Erfindungsgemäß bevorzugt enthält das kosmetische Mittel (M2) daher zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden; (iv) Alkalisierungsmitteln; sowie (v) deren Mischungen.

Es hat sich als vorteilhaft erwiesen, wenn die kosmetischen Mittel (M2) ebenfalls mindestens ein Verdickungsmittel enthalten. Bezüglich dieser Verdickungsmittel bestehen keine prinzipiellen Einschränkungen. Geeignete Verdickungsmittel sind die im Zusammenhang mit dem Vorbehandlungsmittel (M1) angeführten Verbindungen, welche zur Verdickung der Färbemittel (M2) ebenfalls eingesetzt werden können. Daneben können auch die nachfolgend angeführten organischen und anorganischen Verdickungsmittel verwendet werden.

Geeignete Verdickungsmittel sind anionische, synthetische Polymere; kationische, synthetische Polymere sowie nichtionische, synthetische Polymere, wie Polyvinylalkohol oder Polyvinylpyrrolidon.

Die in dem erfindungsgemäßen Verfahren eingesetzten kosmetischen Mittel (M2) können als Verdickungsmittel auch zwitterionische Polymere enthalten, welche ausgewählt sind aus der Gruppe der
- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminiumsalzen und Acrylsäure,
- Copolymere aus N,N,N-Trimethyl-3-[(2-methyl-1-oxo-2-propen-1-yl)amino]-1-propanaminiumsalzen und Methacrylsäure,
- Copolymere aus N,N,N-Trimethyl-2-[(2-methyl-1-oxo-2-propen-1-yl)amino]-1-ethanaminiumsalzen und Acrylsäure,
- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminiumsalzen, Acrylsäure und Acrylamid, z.B. Polyquaternium-53
- Copolymere aus N,N,N-Trimethyl-3-[(2-methyl-1-oxo-2-propen-1-yl)amino]-1-propanaminiumsalzen, Methacrylsäure und Acrylamid,
- Copolymere aus 1-Ethenyl-3-methyl-1H-imidazoliumsalzen, 1-Ethenyl-1H-imidazol, 1-Ethenyl-2-pyrrolidinon und Methacrylsäure, z.B. Polyquaternium-86,
- Copolymere aus 1-Ethenyl-3-methyl-1H-imidazoliumsalzen, 1-Ethenyl-1H-imidazol, 1-Ethenyl-2-pyrrolidinon und Acrylsäure.
Auch Gemische der vorgenannten zwitterionischen Polymere können zur Verdickung der kosmetischen Mittel (M2) eingesetzt werden.

Die erfindungsgemäß in Verfahrensschritt c) verwendeten Färbemittel (M2) können lineare oder verzweigte, gesättigte oder ungesättigte Alkohole mit 8 bis 20 Kohlenstoffatomen enthalten. Es hat sich herausgestellt, dass die zusätzliche Anwesenheit dieser höherkettigen Alkohole das multitonale Färbeergebnis des erfindungsgemäßen Verfahrens noch weiter verbessern kann. Daher ist es bevorzugt, wenn die im erfindungsgemäßen Verfahren eingesetzten kosmetischen Mittel (M2) zusätzlich einen oder mehrere Alkohole aus der Gruppe Arachylalkohol (Eisocan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) enthalten.

Besonders geeignete kosmetische Mittel (M2) enthalten einen oder mehrere höherkettige Alkohole der vorgenannten Gruppe in einer Gesamtmenge von 1,0 bis 10,0 Gew.-%, vorzugsweise von 1,4 bis 8,5 Gew.-%, bevorzugt von 1,8 bis 8,0 Gew.-%, insbesondere von 2,0 bis 7,0 Gew.-%, bezogen auf das Gesamtgewicht des kosmetischen Mittels (M2).

Vorzugsweise werden die Färbemittel (M2) als flüssige Zubereitung bereitgestellt und diesen Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Im Rahmen dieser Ausführungsform kann es vorgesehen sein, dass das anionische Tensid ausgewählt ist aus der Gruppe von Alkylsulfaten und Alkylpolyglykolethersulfaten der Formel R²-O(CH₂-CH₂O)ₓ-OSO₃H, in welcher R² eine lineare Alkylgruppe mit 8 bis 30 Kohlenstoffatomen und x = 0 oder 1 bis 12 ist, Salzen linearer und verzweigter Carbonsäuren mit 8 bis 30 Kohlenstoffatomen, Ethercarbonsäuren der Formel R³-O-(CH₂-CH₂O)ₓ-CH₂-COOH, in welcher R³ eine lineare Alkylgruppe mit 8 bis 30 C-Atomen und x = 0 oder 1 bis 16 ist, sowie deren Mischungen. Die anionischen Tenside werden bevorzugt in einer Gesamtmenge von 0,1 bis 45 Gew.-%, vorzugsweise von 1 bis 30 Gew.-%, insbesondere von 1 bis 15 Gew.-%, bezogen auf die Gesamtmenge des kosmetischen Mittels (M2), eingesetzt.

In diesem Zusammenhang kann es erfindungsgemäß weiterhin vorgesehen sein, dass das nichtionische Tensid ausgewählt ist aus der Gruppe von ethoxylierten Alkoholen und Carbonsäuren mit 8 bis 13 Kohlenstoffatomen und 2 bis 30 Ethylenoxideinheiten, Anlagerungsprodukten von 5 bis 60 Mol Ethylenoxid an Rizinusöl und gehärtetes Rizinusöl, Alkylpolyglucosiden der Formel R¹O-[G]ₚ, in welcher R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, Monoethanolamiden von Carbonsäuren 8 bis 30 Kohlenstoffatomen sowie deren Mischungen.

Darüber hinaus kann es im Rahmen dieser Ausführungsform auch vorgesehen sein, dass das amphotere Tensid ausgewählt ist aus der Gruppe von Amphoacetaten mit Carbonsäureresten mit 8 bis 30 Kohlenstoffatomen, N-Alkylglycinen, N-Alkylpropionsäuren, N-Alkylamidobuttersäuren, N-Alkyliminodipropionsäuren, N-Hydroxyethyl-N-alkylamidopropylglycinen, N-Alkyltaurinen, N-Alkylsarcosinen, 2-Alkylaminopropionsäuren, Alkylaminoessigsäuren sowie deren Mischungen.

Im Rahmen dieser Ausführungsform kann es zudem vorgesehen sein, dass das zwitterionische Tensid ausgewählt ist aus der Gruppe von Betainen, N-Alkyl-N,N-diemthylammoniumglycinaten, N-Acylamidopropyl-N,N-dimethylammoniumglycinaten, 2-Alkyl-3-carboxymethyl-3-hydroxyethyl-imidazolinen sowie deren Mischungen.

Die nichtionischen und/oder zwitterionischen und/oder amphoteren Tenside werden bevorzugt in einer Gesamtmenge von 0,1 bis 45 Gew.-%, vorzugsweise von 1 bis 30 Gew.-%, insbesondere von 1 bis 15 Gew.-%, bezogen auf die Gesamtmenge des kosmetischen Mittels (M2), eingesetzt.

Auch das kosmetische Mittel (M2) kann mindestens ein Alkalisierungsmittel enthalten. Geeignete Alkalisierungsmittel sowie deren einsetzbare Gesamtmengen sind bereits im Zusammenhang mit dem Vorbehandlungsmittel (M1) angeführt worden. Die Einstellung eines basischen pH-Werts unter Verwendung des mindestens einen Alkalisierungsmittels ist erforderlich, um eine Öffnung der äußeren Schuppenschicht (Cuticula) zu gewährleisten und eine Penetration der Oxidationsfarbstoffvorprodukte in das Haar zu ermöglichen.

Erfindungsgemäß bevorzugte Verfahren sind dadurch gekennzeichnet, dass das kosmetische Mittel (M2) einen pH-Wert von pH 9,2 bis pH 10,5, aufweist.

Um eine lebendige multitonale Färbung zu erzielen, ist es von Vorteil, wenn es bei dem sequentiellen Auftragen der kosmetischen Mittel (M1) und (M2) nicht zu starken pH-Wertschwankungen kommt, da hierdurch eine nur unzureichende Penetration der keratinischen Fasern und damit auch ein verschlechtertes Färbeergebnis auftreten können. Erfindungsgemäße Verfahren sind daher dadurch gekennzeichnet, dass das kosmetischen Mittel (M1) und das kosmetischen Mittel (M2) identische pH-Werte aufweisen.

Die Oxidationsfarbstoffvorprodukte (Entwickler und Kuppler) selbst sind nicht gefärbt. Die Bildung der eigentlichen Farbstoffe erfolgt erst im Verlauf der Anwendung durch den Kontakt der Oxidationsfarbstoffvorprodukte mit einem Oxidationsmittel (vorzugsweise Wasserstoffperoxid). In einer chemischen Reaktion werden die als Oxidationsfarbstoffvorprodukte eingesetzten Entwickler (wie beispielsweise p-Phenylendiamin-Derivate oder p-Aminophenolderivate) durch Wasserstoffperoxid zunächst oxidativ in eine reaktive Zwischenstufe, auch Chinonimin oder Chinondiimin genannt, überführt, welche dann in einer oxidativen Kupplungsreaktion mit den Kupplern zum jeweiligen Farbstoff reagiert.

Die kosmetischen Mittel (M2) enthalten daher zusätzlich ein oder mehrere Oxidationsmittel (M2-2). Als Oxidationsmittel kommen Persulfate, Peroxodisulfate, Chlorite, Hypochlorite und insbesondere Wasserstoffperoxid und/oder eines seiner festen Anlagerungsprodukte an organische oder anorganische Verbindungen in Frage.

Erfindungsgemäß bevorzugte Verfahren sind daher dadurch gekennzeichnet, dass das kosmetische Mittel (M2) mindestens ein Oxidationsmittel (M2-2) aus der Gruppe von Persulfaten, Peroxodisulfaten, Chloriten, Hypochloriten, Wasserstoffperoxid sowie dessen feste Anlagerungsprodukte an Harnstoff, Melamin, Polyvinylpyrrolidon und Natriumborat, bevorzugt Wasserstoffperoxid, in einer Gesamtmenge von 0,5 bis 10 Gew.-%, vorzugsweise von 1,0 bis 10 Gew.-%, insbesondere von 1,5 bis 10 Gew.-%, bezogen auf das Gesamtgewicht des kosmetischen Mittels (M2), enthält. Falls als Oxidationsmittel Wasserstoffperoxid sowie dessen feste Anlagerungsprodukte eingesetzt werden, berechnet sich die vorstehend genannte Gesamtmenge auf 100%iges H₂O₂.

In einer weiteren bevorzugten Ausführungsform ist das kosmetische Mittel (M2) ein Mittel zum Färben und gegebenenfalls gleichzeitigen Aufhellen keratinischer Fasern, welches Wasserstoffperoxid in einer Gesamtmenge von 0,5 bis 15 Gew.-%, vorzugsweise 1 bis 12,5 Gew.-%, bevorzugt 1,5 bis 10 Gew.-%, insbesondere 1,5 bis 6,5 Gew.-%, bezogen auf das Gesamtgewicht des kosmetischen Mittels (M2), enthält. Die vorstehend genannte Gesamtmenge an Wasserstoffperoxid ist hierbei auf 100%iges H₂O₂ bezogen.

Zur Erzielung einer verstärkten Aufhell- und Bleichwirkung kann das kosmetische Mittel (M2) weiterhin mindestens ein Peroxo-Salz enthalten. Geeignete Peroxo-Salze sind anorganische Peroxoverbindungen, bevorzugt ausgewählt aus der Gruppe von Ammoniumperoxodisulfat, Alkalimetallperoxodisulfaten, Ammoniumperoxomonosulfat, Alkalimetallperoxomonosulfaten, Alkalimetallperoxodiphosphaten, Erdalkalimetallperoxiden sowie deren Mischungen. Besonders bevorzugt sind Peroxodisulfate, insbesondere Ammoniumperoxodisulfat, Kaliumperoxodisulfat und Natriumperoxodisulfat.

Die zuvor genannten Peroxosalze sind in einer Gesamtmenge von 0,5 bis 20 Gew.-%, vorzugsweise 1,0 bis 12,5 Gew.-%, bevorzugt 2,5 bis 10 Gew.-%, insbesondere 3,0 bis 6,0 Gew.-%, bezogen auf das Gesamtgewicht des kosmetischen Mittels (M2), enthalten.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht. Üblicherweise werden oxidative Färbemittel daher in Form eines aus zwei Komponenten bestehenden "Kits" (Mehrkomponenten-Verpackungseinheit) angeboten, wobei die erste Komponente die Oxidationsfarbstoffvorprodukte und gegebenenfalls direktziehenden Farbstoffe sowie ein Alkalisierungsmittel (beispielsweise Ammoniak) und die zweite Komponente das Oxidationsmittel enthält.

In einer weiteren Ausführungsform der vorliegenden Erfindung sind daher kosmetische Mittel (M2) bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden und wobei ein Container ein Färbemittel (M2a), welches in einem kosmetischen Träger mindestens ein Oxidationsfarbstoffvorprodukt enthält, und ein weiterer Container eine Oxidationsmittelzubereitung (M2b), enthaltend mindestens ein Oxidationsmittel, enthält.

Das Färbemittel (M2a) enthält hierbei bevorzugt die zuvor im Zusammenhang mit dem kosmetischen Mittel (M2) aufgeführten Oxidationsfarbstoffvorprodukte vom Entwicklertyp und/oder Kupplertyp, gegebenenfalls mindestens einen direktziehenden Farbstoff und gegebenenfalls mindestens einen zuvor im Zusammenhang mit dem kosmetischen Mittel (M2) aufgeführten Wirk-, Hilfs- oder Zusatzstoff. Die Oxidationsmittelzubereitung (M2b) enthält bevorzugt ein Oxidationsmittel in Form von Wasserstoffperoxid und/oder eines seiner festen Anlagerungsprodukte an organische oder anorganische Verbindungen, wie Harnstoff, Melamin sowie Natriumborat.

Solche Oxidationsmittelzubereitungen (M2b) sind vorzugsweise wässrige, fließfähige Oxidationsmittelzubereitungen. Dabei sind bevorzugte Zubereitungen (M2b) dadurch gekennzeichnet, dass die fließfähige Oxidationsmittelzubereitung (M2b) 40 bis 90 Gew.-%, vorzugsweise 50 bis 90 Gew.-%, bevorzugt 55 bis 89 Gew.-%, weiter bevorzugt 60 bis 87 Gew.-%, insbesondere 65 bis 85 Gew.-%, bezogen auf das Gesamtgewicht der Oxidationsmittelzubereitung (M2b), Wasser enthält.

Bevorzugt beträgt die Gesamtmenge an Oxidationsmittel, insbesondere Wasserstoffperoxid, in der Oxidationsmittelzubereitung (M2b) 0,5 bis 12 Gew.-%, vorzugsweise 2,0 bis 10 Gew.-%, insbesondere 1,5 bis 6,0 Gew.-%, bezogen auf das Gesamtgewicht der Oxidationsmittelzubereitung (M2b). Falls als Oxidationsmittel Wasserstoffperoxid sowie dessen feste Anlagerungsprodukte eingesetzt werden, berechnet sich die vorstehend genannte Gesamtmenge auf 100%iges H₂O₂.

Erfindungsgemäß kann die Oxidationsmittelzubereitung (M2b) auch zusammen mit einem Katalysator auf das Haar aufgebracht werden, welcher die Oxidation der Farbstoffvorprodukte aktiviert. Solche Katalysatoren sind z. B. bestimmte Enzyme, lodide, Chinone oder Metallionen.

Es hat sich als vorteilhaft erwiesen, wenn die Oxidationsmittelzubereitungen (M2b) zur Stabilisierung des Oxidationsmittels, insbesondere des Wasserstoffperoxids, zusätzlich mindestens einen Stabilisator oder Komplexbildner enthalten. Besonders bevorzugte Stabilisatoren sind insbesondere EDTA und EDDS sowie Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

## Patentansprüche

1. Verfahren zum oxidativen Färben von keratinischen Fasern, wobei das Verfahren die folgenden Verfahrensschritte in der angegebenen Reihenfolge umfasst:
a) Applikation eines kosmetischen Mittels (M1) auf die keratinischen Fasern, wobei das kosmetische Mittel (M1) nur auf einzelne Strähnchen appliziert wird,
b) Einwirkenlassen des Mittels (M1) für einen Zeitraum von 30 Sekunden bis 40 Minuten auf den keratinischen Fasern,
c) Applikation eines kosmetischen Mittels (M2) auf die mit dem kosmetischen Mittel (M1) beaufschlagten keratinischen Fasern,
d) Einwirkenlassen der kosmetischen Mittel (M1) und (M2) für einen Zeitraum von 1 bis 70 Minuten auf den keratinischen Fasern,
e) Ausspülen der kosmetischen Mittel (M1) und (M2),
**dadurch gekennzeichnet, dass**
- das kosmetische Mittel (M1)
mindestens einen direktziehenden Farbstoff enthält (M1-1), und
mindestens ein Verdickungsmittel enthält (M1-2),
keine Oxidationsfarbstoffvorprodukte enthält (M1-3),
einen pH-Wert von 9,2 bis 10,5 aufweist,
eine dynamische Viskosität von 10.000 bis 50.000 mPa*s, gemessen mit Brookfield RDV II+, Spindel Nr. 4, 4 rpm, 20 °C, aufweist,
- das kosmetische Mittel (M2)
mindestens ein Oxidationsfarbstoffvorprodukt enthält (M2-1), und
mindestens ein Oxidationsmittel enthält (M2-2),
- das kosmetischen Mittel (M1) und das kosmetischen Mittel (M2) identische pH-Werte aufweisen.

2. Verfahren nach Anspruch 1, **dadurch gekennzeichnet, dass** das kosmetische Mittel (M1) in Verfahrensschritt b) für einen Zeitraum von 30 Sekunden bis 30 Minuten, vorzugsweise von 30 Sekunden bis 20 Minuten, bevorzugt von 30 Sekunden bis 18 Minuten, insbesondere von 30 Sekunden bis 15 Minuten, auf den keratinischen Fasern Einwirken gelassen wird.

3. Verfahren nach einem der vorangehenden Ansprüche, **dadurch gekennzeichnet, dass** die kosmetischen Mittel (M1) und (M2) in Verfahrensschritt d) für einen Zeitraum von 1 bis 60 Minuten, vorzugsweise von 5 bis 50 Minuten, insbesondere von 10 bis 45 Minuten, Einwirken gelassen werden.

4. Verfahren nach einem der vorangehenden Ansprüche, **dadurch gekennzeichnet, dass** das kosmetische Mittel (M1) den mindestens einen direktziehenden Farbstoff (M1-1) in einer Gesamtmenge von 0,0001 bis 5,5 Gew.-%, vorzugsweise von 0,0005 bis 5,0 Gew.-%, bevorzugt von 0,001 bis 4,5 Gew.-%, weiter bevorzugt von 0,001 bis 4,0 Gew.-%, insbesondere von 0,001 bis 3,0 Gew.-%, bezogen auf das Gesamtgewicht des kosmetischen Mittels (M1), enthält.

5. Verfahren nach einem der vorangehenden Ansprüche, **dadurch gekennzeichnet, dass** das mindestens eine Verdickungsmittel (M1-2) ausgewählt ist aus der Gruppe von Cellulose, Xanthan, vernetzten Homo- oder Copolymeren der Acrylsäure, der Methacrylsäure und deren Salzen, vernetzten Copolymeren der ethoxylierten Alkylester der Methacrylsäure und der sulfonierten Acrylsäureamide sowie deren Salzen vernetzten Copolymeren der Methacrylsäure, der Acrylsäureamide und der sulfonierten Acrylsäureamide sowie deren Salzen und Mischungen dieser Verdickungsmittel.

6. Verfahren nach einem der vorangehenden Ansprüche, **dadurch gekennzeichnet, dass** das kosmetische Mittel (M1) das mindestens eine Verdickungsmittel (M1-2) in einer Gesamtmenge von 0,1 bis 5,0 Gew.-%, vorzugsweise von 0,3 bis 4,5 Gew.-%, bevorzugt von 0,5 bis 4,0 Gew.-%, weiter bevorzugt von 0,7 bis 3,5 Gew.-%, insbesondere von 0,8 bis 3,0 Gew.-%, bezogen auf das Gesamtgewicht des kosmetischen Mittels (M1), enthält.

7. Verfahren nach einem der vorangehenden Ansprüche, **dadurch gekennzeichnet, dass** das kosmetische Mittel (M2) als Oxidationsfarbstoffvorprodukt (M2-1) mindestens ein Oxidationsfarbstoffvorprodukt vom Entwickler- und/oder Kupplertyp enthält.

8. Verfahren nach einem der vorangehenden Ansprüche, **dadurch gekennzeichnet, dass** das kosmetische Mittel (M2) mindestens ein Oxidationsmittel (M2-2) aus der Gruppe von Persulfaten, Peroxodisulfaten, Chloriten, Hypochloriten, Wasserstoffperoxid sowie dessen feste Anlagerungsprodukte an Harnstoff, Melamin, Polyvinylpyrrolidon und Natriumborat, bevorzugt Wasserstoffperoxid, in einer Gesamtmenge von 0,5 bis 10 Gew.-%, vorzugsweise von 1,0 bis 10 Gew.-%, insbesondere von 1,5 bis 10 Gew.-%, bezogen auf das Gesamtgewicht des kosmetischen Mittels (M2), enthält.

## Claims

1. A method for oxidatively dyeing keratin fibers, the method comprising the following method steps in the sequence indicated:
a) applying a cosmetic agent (M1) to the keratin fibers, the cosmetic agent (M1) being applied only to individual strands,
b) allowing the agent (M1) to act on the keratin fibers for a period of from 30 seconds to 40 minutes,
c) applying a cosmetic agent (M2) to the keratin fibers that have been subjected to the cosmetic agent (M1),
d) allowing the cosmetic agents (M1) and (M2) to act on the keratin fibers for a period of from 1 to 70 minutes,
e) rinsing off the cosmetic agents (M1) and (M2),
**characterized in that**
- the cosmetic agent (M1)
contains at least one direct dye (M1-1), and
contains at least one thickening agent (M1-2),
does not contain any oxidation dye precursors (M1-3),
has a pH of from 9.2 to 10.5,
has a dynamic viscosity of from 10,000 to 50,000 mPa*s, measured using Brookfield RDV II+, spindle no. 4, 4 rpm, 20 °C,
- the cosmetic agent (M2)
contains at least one oxidation dye precursor (M2-1), and
contains at least one oxidizing agent (M2-2),
- and the cosmetic agent (M1) and the cosmetic agent (M2) have identical pH values.

2. The method according to claim 1, **characterized in that** in method step b), the cosmetic agent (M1) is allowed to act on the keratin fibers for a period of from 30 seconds to 30 minutes, preferably from 30 seconds to 20 minutes, more preferably from 30 seconds to 18 minutes, in particular from 30 seconds to 15 minutes.

3. The method according to one of the preceding claims, **characterized in that** in method step d), the cosmetic agents (M1) and (M2) are allowed to act for a period of from 1 to 60 minutes, preferably from 5 to 50 minutes, in particular from 10 to 45 minutes.

4. The method according to one of the preceding claims, **characterized in that** the cosmetic agent (M1) contains the at least one direct dye (M1-1) in a total amount of from 0.0001 to 5.5 wt.%, preferably from 0.0005 to 5.0 wt.%, more preferably from 0.001 to 4.5 wt.%, even more preferably from 0.001 to 4.0 wt.%, in particular from 0.001 to 3.0 wt.%, based on the total weight of the cosmetic agent (M1).

5. The method according to one of the preceding claims, **characterized in that** the at least one thickening agent (M1-2) is selected from the group of cellulose, xanthan gum, crosslinked homo- or copolymers of acrylic acid, of methacrylic acid and the salts thereof, crosslinked copolymers of ethoxylated alkyl esters of methacrylic acid and of sulfonated acrylic acid amides and the salts thereof, crosslinked copolymers of methacrylic acid, of acrylic acid amides and of sulfonated acrylic acid amides and the salts thereof, and mixtures of these thickening agents.

6. The method according to one of the preceding claims, **characterized in that** the cosmetic agent (M1) contains the at least one thickening agent (M1-2) in a total amount of from 0.1 to 5.0 wt.%, preferably from 0.3 to 4.5 wt.%, more preferably from 0.5 to 4.0 wt.%, even more preferably from 0.7 to 3.5 wt.%, in particular from 0.8 to 3.0 wt.%, based on the total weight of the cosmetic agent (M1).

7. The method according to one of the preceding claims, **characterized in that** the cosmetic agent (M2) contains at least one developer-type and/or coupler-type oxidation dye precursor as the oxidation dye precursor (M2-1).

8. The method according to one of the preceding claims, **characterized in that** the cosmetic agent (M2) contains at least one oxidizing agent (M2-2) from the group of persulfates, peroxydisulfates, chlorites, hypochlorites, hydrogen peroxide and the solid addition products thereof to urea, melamine, polyvinylpyrrolidone and sodium borate, preferably hydrogen peroxide, in a total amount of from 0.5 to 10 wt.%, preferably from 1.0 to 10 wt.%, in particular from 1.5 to 10 wt.%, based on the total weight of the cosmetic agent (M2).

## Revendications

1. Procédé de coloration par oxydation de fibres kératiniques, le procédé comprenant les étapes de procédé suivantes, dans l'ordre indiqué :
a) l'application d'un agent cosmétique (M1) sur les fibres kératiniques, l'agent cosmétique (M1) n'étant appliqué que sur des mèches individuelles,
b) le fait de laisser agir l'agent (M1) sur les fibres kératiniques pendant une durée de 30 à 40 minutes,
c) l'application d'un agent cosmétique (M2) sur les fibres kératiniques sur lesquelles agit l'agent cosmétique (M1),
d) le fait de laisser agir les agents cosmétiques (M1) et (M2) sur les fibres kératiniques pendant une durée de 1 à 70 minutes,
e) le rinçage des produits cosmétiques (M1) et (M2),
**caractérisé en ce que**
- l'agent cosmétique (M1)
contient au moins un colorant direct (M1-1), et
contient au moins un agent épaississant (M1-2),
ne contient aucun précurseur de colorant d'oxydation (M1-3),
présente un pH de 9,2 à 10,5,
présente une viscosité dynamique de 10 000 à 50 000 mPa*s, mesurée avec Brookfield RDV II+,
broche n° 4, 4 tr/min, 20 °C,
- l'agent cosmétique (M2)
contient au moins un précurseur de colorant d'oxydation (M2-1), et
au moins un agent oxydant (M2-2),
- l'agent cosmétique (M1) et l'agent cosmétique (M2) présentent des valeurs de pH identiques.

2. Procédé selon la revendication 1, **caractérisé en ce qu'**on laisse agir l'agent cosmétique (M1) sur les fibres kératiniques dans l'étape de procédé b) pendant une durée de 30 secondes à 30 minutes, de préférence de 30 secondes à 20 minutes, de manière préférée de 30 secondes à 18 minutes, en particulier de 30 secondes à 15 minutes.

3. Procédé selon l'une quelconque des revendications précédentes, **caractérisée en ce qu'**on laisse agir les agents cosmétiques (M1) et (M2) dans l'étape de procédé d) pendant une durée de 1 à 60 minutes, de préférence de 5 à 50 minutes, en particulier de 10 à 45 minutes.

4. Procédé selon l'une quelconque des revendications précédentes, **caractérisée en ce que** l'agent cosmétique (M1) contient l'au moins un colorant direct (M1-1) en une quantité totale de 0,0001 à 5,5 % en poids, de préférence de 0,0005 à 5,0 % en poids, de manière préférée de 0,001 à 4,5 % en poids, de manière davantage préférée de 0,001 à 4,0 % en poids, en particulier de 0,001 à 3,0 % en poids, par rapport au poids total de l'agent cosmétique (M1).

5. Procédé selon l'une quelconque des revendications précédentes, **caractérisée en ce que** l'au moins un agent épaississant (M1-2) est choisi dans le groupe constitué de la cellulose, de la gomme xanthane, des homo- ou copolymères réticulés d'acide acrylique, de l'acide méthacrylique et de leurs sels, des copolymères réticulés d'esters alkyliques éthoxylés d'acide méthacrylique et d'amides d'acide acrylique sulfonés et de leurs sels, des copolymères réticulés d'acide méthacrylique, d'amides d'acide acrylique et d'amides d'acide acrylique sulfonés et de leurs sels et des mélanges de ces agents épaississants.

6. Procédé selon l'une quelconque des revendications précédentes, **caractérisée en ce que** l'agent cosmétique (M1) contient l'au moins un agent épaississant (M1-2) en une quantité totale allant de 0,1 à 5,0 % en poids, de préférence de 0,3 à 4,5 % en poids, de manière préférée de 0,5 à 4,0 % en poids, de manière encore plus préférée de 0,7 à 3,5 % en poids, en particulier de 0,8 à 3,0 % en poids, par rapport au poids total de l'agent cosmétique (M1).

7. Procédé selon l'une quelconque des revendications précédentes, **caractérisée en ce que** l'agent cosmétique (M2) contient, comme précurseur de colorant d'oxydation (M2-1), au moins un précurseur de colorant d'oxydation du type révélateur et/ou coupleur.

8. Procédé selon l'une quelconque des revendications précédentes, **caractérisée en ce que** l'agent cosmétique (M2) contient au moins un agent oxydant (M2-2) du groupe constitué des persulfates, des peroxodisulfates, des chlorites, des hypochlorites, du peroxyde d'hydrogène et de ses produits d'addition solides sur l'urée, de la mélamine, de la polyvinylpyrrolidone et du borate de sodium, de manière préférée du peroxyde d'hydrogène, en une quantité totale de 0,5 à 10 % en poids, de préférence de 1,0 à 10 % en poids, en particulier de 1,5 à 10 % en poids, par rapport au poids total de l'agent cosmétique (M2).
